# EUROPEAN PATENT APPLICATION

(11) **EP 4 603 138 A1**
(43) Date of publication of application: **20.08.2025**
(21) Application number: 23880204.5
(22) Date of filing: 18.10.2023
(51) Int. Cl.: A61N 5/10

(54) **MULTI-GANTRY LOW-DOSE RADIATION APPARATUS**

(30) Priority: 19.10.2022 KR 20220135106; 13.09.2023 KR 20230121982
(71) Applicant: ReadyCure Inc., Seoul 02792 (KR)
(72) Inventor: CHUNG, Weon Kuu, Seoul 06288 (KR)
(74) Representative: Prüfer & Partner mbB Patentanwälte · Rechtsanwälte
(86) International application number: PCT/KR2023/016099
(87) International publication number: WO 2024/085627

(57) **Abstract**

According to the present invention, the present invention may provide a multi-gantry low-dose radiation apparatus including a main body having a bed on which a patient lies and receives treatment, wherein the bed is formed longitudinally on the main body; radiation gantries installed to radiate a plurality of low-dose radiation toward a patient's body from the inner periphery of a ring gate while moving along rails on the upper side of the main body; a gantry mounting portion formed at one end of the main body to accommodate the radiation gantries in an operational standby state; and a controller installed on one side of the gantry mounting portion to check the condition of a patient and set a treatment direction using entered patient information and control the operation of the radiation gantries, wherein the radiation gantries are installed in multiple numbers along the length of the main body and are driven in conjunction with each other or individually.

## Description

### [Cross-Reference to Related Applications]

This application is a National Stage Entry of PCT International Application No. PCT/KR2023/016099, which was filed on October 18, 2023, and claims priority to Korean Patent Application No. 10-2022-0135106, filed on October 19, 2022, and Korean Patent Application No. 10-2023-0121982, filed on September 13, 2023, in the Korean Intellectual Property Office, the disclosures of each of which are incorporated herein by reference.

### [Technical Field]

The present invention relates to a multi-gantry low-dose radiation apparatus, and more particularly, to a multi-gantry low-dose radiation apparatus capable of being used continuously without a break period due to heat generated when emitting radiation beams by including multiple gantries.

### [Background Art]

Generally, therapeutic radiation used for medical purposes is applied to the tumors of cancer patients to kill cancer cells by preventing the cancer cells from reproducing any further, or to relieve the patient's pain.

Recently, the intensity-modulated radiation therapy (IMRT) method has been in the spotlight. According to this method, by modulating the intensity of radiation using a multi-leaf collimator (MLC) to suit the shape, size, and location of tumors and irradiating radiation adjusted to the optimal energy, side effects of radiation on normal tissues can be reduced and the therapeutic effect can be maximized.

However, there was a problem that the treatment devices used in such radiation therapy were limited to cancer treatment.

In addition, the conventional radiation apparatuses had a problem in that the radiation apparatuses could not emit radiation for a long time due to heat generated when a radiation source emits radiation beams.

In addition, the conventional radiation apparatuses had a problem in that the radiation apparatuses could not emit a sufficient amount of radiation beams necessary for patient treatment due to the limited number of radiation sources used.

### [Disclosure]

### [Technical Problem]

Therefore, the present invention has been made in view of the above problems, and it is one object of the present invention to provide a multi-gantry low-dose radiation apparatus capable of being used continuously without a break period due to heat generated when emitting radiation beams by including multiple gantries.

It is another object of the present invention to provide a multi-gantry low-dose radiation apparatus that enables low-dose radiation beams to be radiated alternately or simultaneously to a patient's body by allowing a plurality of gantries to be individually driven.

It is still another object of the present invention to minimize patient discomfort by minimizing waiting time for a patient in the radiation apparatus.

It is still another object of the present invention to meet the total radiation emission required for treatment by increasing the number of carbon nanotube light sources emitting radiation beams in a multi-gantry manner.

It is still another object of the present invention to provide a variety of treatment options by irradiating multiple parts of the patient's body with radiation beams simultaneously by individually controlling the emission positions of radiation beams from a plurality of gantries while moving along rails, or by moving the radiation beams to different parts of the body.

It is still another object of the present invention to minimize cell damage to a treatment area due to radiation exposure by installing pulsed beam-type carbon nanotube light sources around the inner periphery of a gantry and controlling the radiation beam irradiation time (beam on-off time) and the radiation beam irradiation interval between carbon nanotube light sources to the cell damage repair time (DNA repair time) that allows DNA damage repair in normal cells.

It is still another object of the present invention to provide an optimized radiation treatment environment by adjusting the energy size, angle, and dose of radiation radiated from the radiation apparatus according to treatment purposes and individual patient characteristics.

It is yet another object of the present invention to provide an open radiation apparatus that is capable of radiating a uniform beam to a target object such as the head (brain) area by increasing the number of carbon nanotube light sources that emit radiation beams by a multi-gantry method and is safe for the human body by emitting low-dose, low-energy radiation beams through multiple carbon nanotube light sources.

### [Technical Solution]

In accordance with one aspect of the present invention, provided is a multi-gantry low-dose radiation apparatus including a main body having a bed on which a patient lies and receives treatment, wherein the bed is formed longitudinally on the main body; radiation gantries installed to radiate a plurality of low-dose radiation toward a patient's body from an inner periphery of a ring gate while moving along rails on an upper side of the main body; a gantry mounting portion formed at one end of the main body to accommodate the radiation gantries in an operational standby state; and a controller installed on one side of the gantry mounting portion to check a condition of a patient and set a treatment direction using entered patient information and control operation of the radiation gantries, wherein the radiation gantries are installed in multiple numbers along a length of the main body and are driven in conjunction with each other or individually.

In addition, the bed on which a patient lies and receives treatment may be formed longitudinally on an upper part of the main body, the main body may be provided with an open structure, and rails may be formed on sides of the main body to guide movement of the radiation gantries.

In addition, a support may be formed on one lower side of the main body to secure the main body in a state of floating at a certain height from a floor of a building. The support may be installed in a biased position on one lower side of the main body, allowing free movement of the radiation gate to an opposite side where the support is not installed.

In addition, the radiation gantries may be installed in multiple numbers in a longitudinal direction of the main body, and the radiation gantries may be driven in conjunction with each other or individually.

In addition, the radiation gantries may form a ring gate that surrounds a body of a patient, a plurality of carbon nanotube light sources that radiate therapeutic radiation toward the body of the patient may be formed at an inner periphery of the ring gate, and the carbon nanotube light sources emitting low-energy of 100 to 120, 120'kVp (kilovolt peak) and low-dose radiation of 10 to 100 cGy/min may be distributed according to bands.

In addition, the carbon nanotube light sources placed at a center of the radiation gantries may emit the highest energy, the carbon nanotube light sources placed toward both ends centered on the center of the radiation gantries may emit progressively lower energy, and the carbon nanotube light sources may be arranged in a left-right symmetrical structure.

In addition, carbon nanotube light sources may be formed in multiple arrays on a gate inner periphery of the radiation gantries, and each array of carbon nanotube light sources may be operated alternately.

In addition, the gantry mounting portion may be provided as a ring-shaped structure that surrounds an outer surface of the radiation gantries to accommodate the radiation gantries in an operational standby state, and a table for installing a controller may be formed at an open end thereof.

In addition the gantry mounting portion may be electrically and mechanically or electrically connected to the main body, and may be formed as an independent structure that is mechanically separated from the main body.

In addition, the controller may include a monitor for monitoring patient information and an input device for entering commands to control the apparatus.

In addition, the controller may be provided as a touch panel.

### [Advantageous effects]

According to the present invention, by installing multiple gantries, continuous use is possible without a break period due to heat generated when emitting radiation beams. **In** addition, by allowing the gantries to be driven individually, the patient's body can be irradiated with low-dose radiation beams alternately or simultaneously.

The present invention can minimize patient discomfort by minimizing waiting time for a patient in the radiation apparatus.

The present invention can increase efficiency by meeting the total radiation emission required for treatment by increasing the number of carbon nanotube light sources emitting radiation beams in a multi-gantry manner.

The present invention can provide a variety of treatment options by irradiating multiple parts of the patient's body with radiation beams simultaneously by individually controlling the emission positions of radiation beams from a plurality of gantries while moving along rails, or by moving the radiation beams to different parts of the body.

The present invention can provide an optimized radiation treatment environment by adjusting the energy size, angle, and dose of radiation radiated from the radiation apparatus according to treatment purposes and individual patient characteristics.

According to the present invention, a target object such as the head (brain) area can be irradiated with a uniform beam by increasing the number of carbon nanotube light sources that emit radiation beams by a multi-gantry method. In addition, an environment that is efficient and safe for human body can be provided by emitting low-dose, low-energy radiation beams through multiple carbon nanotube light sources.

### [Description of Drawings]

FIG. 1 is a perspective view showing a multi-gantry low-dose radiation apparatus according to the present invention.
FIG. 2 is a side view illustrating a multi-gantry low-dose radiation apparatus according to the present invention.
FIG. 3 is an example of the gantry operation status of a multi-gantry low-dose radiation apparatus according to the present invention.
FIG. 4 illustrates the arrangement of carbon nanotube light sources in a multi-gantry low-dose radiation apparatus according to the present invention.
FIG. 5 is a graph showing the progress of concurrent drug treatment and radiation treatment performed on experimental mice according to the conventional method.
FIG. 6 is a graph showing the progress of concurrent treatment on experimental mice using the low-dose radiation apparatus of the present invention.
FIG. 7 is a graph comparing the Aβ cortical image and the number of plaques per unit area according to the results of radiation treatment using the conventional method with the control group.
FIG. 8 is a graph comparing the Aβ cortical image and the number of plaques per unit area according to the results of low-dose radiation treatment of the present invention with the control group.
FIG. 9 is a graph showing the relative expression of radiation treatment results according to the conventional method compared to the control group through RT-PCR.
FIG. 10 is a graph showing the relative expression of low-dose radiation treatment results according to the present invention compared to the control group through RT-PCR.

### [Best Mode]

Hereinafter, embodiments will be described in detail with reference to the attached drawings. However, the scope of the patent application is not limited or restricted by these embodiments. The same reference numerals presented in each drawing represent the same components.

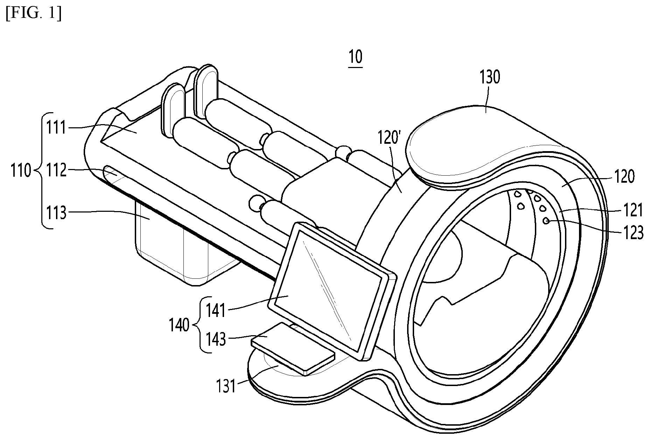

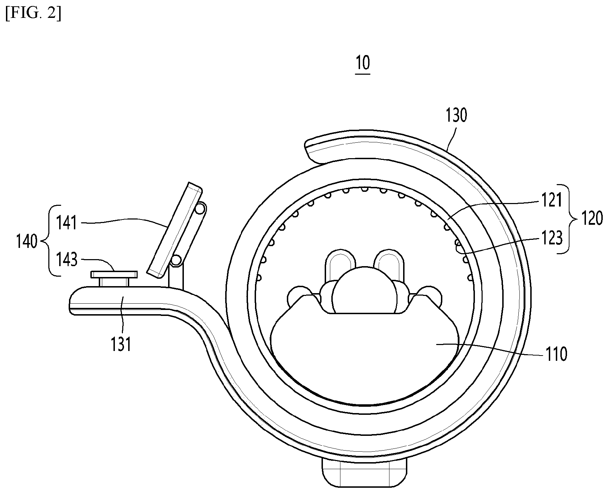

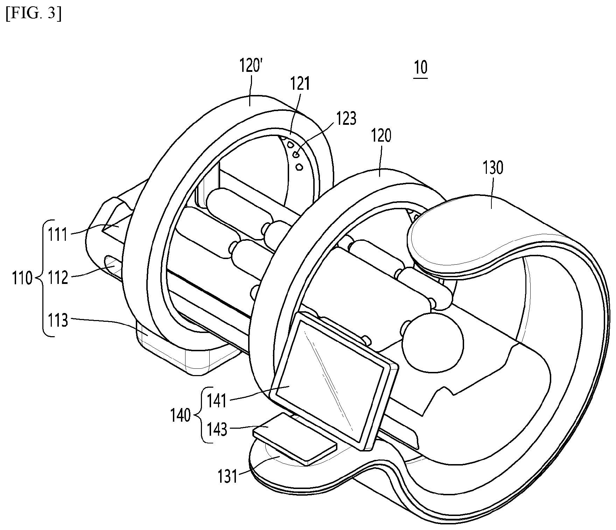

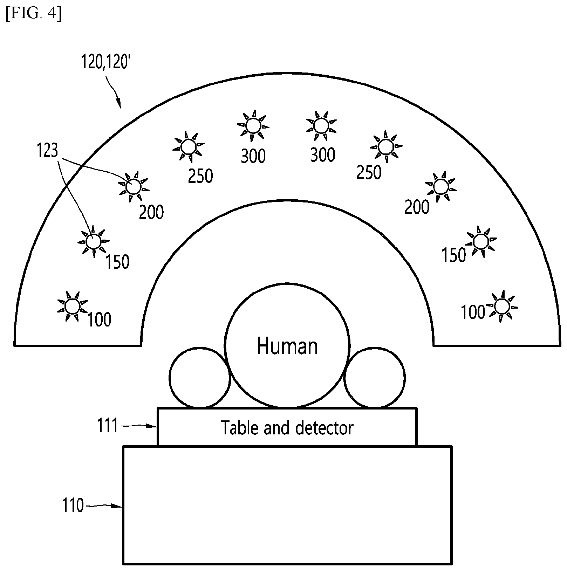

FIG. 1 is a perspective view showing a multi-gantry low-dose radiation apparatus according to the present invention, FIG. 2 is a side view illustrating a multi-gantry low-dose radiation apparatus according to the present invention, FIG. 3 is an example of the gantry operation status of a multi-gantry low-dose radiation apparatus according to the present invention, and FIG. 4 illustrates the arrangement of carbon nanotube light sources in a multi-gantry low-dose radiation apparatus according to the present invention.

Referring to FIGS. 1 to 3, a low-dose radiation apparatus 10 according to the present invention may include a main body 110, radiation gantries 120 and 120', a gantry mounting portion 130, and a controller 140.

On the upper part of the main body 110, a bed 111 may be formed longitudinally, on which a patient lies and receives treatment.

At this time, the main body 110 is provided with an open structure, which may provide an environment in which a patient may communicate with the outside world. This open structure may eliminate patients' fear of confinement and dispel concerns about radiation exposure.

On the side of the main body 110, rails 112 may be formed to guide the movement of the radiation gantries 120 and 120'.

The rails 112 may be installed on the transport path of the radiation gantries 120 and 120' and may support the lower portions of the radiation gantries 120 and 120' in a sliding manner.

The rails 112 may be installed on the longitudinal left and right sides of the main body 110, respectively.

The rails 112 may be composed of fixed rails (not shown) arranged inside the main body 110, rail guides (not shown) coupled to and sliding on the fixed rails, and driving devices (not shown) that reciprocate the rail guides in the rail direction. At this time, the actuator may use a motor and an actuator such as a hydraulic cylinder as a power source.

The rail guides are combined with the radiation gantries 120 and 120' so that the radiation gantries 120 and 120' may be reciprocally driven together with the rail guides when the driving devices are operated.

At this time, the number of rail guides and driving devices may vary depending on the number of radiation gantries being installed.

In addition, a radiation detector (not shown) may be formed inside the main body 110. The radiation detector is a detection device that detects radiation that has passed through a subject (patient).

In addition, signals detected by the radiation detector may be provided as an image or screen through a monitor 141 of the controller 140 to be described later.

In addition, a support 113 may be formed on one side of the lower portion of the main body 110 to fix the main body 110 in a state of floating at a certain height from the floor of a building. The support 113 may have electrical equipment, control equipment, and communication equipment installed therein for operating the ionization radiation apparatus.

At this time, the support 113 may be installed so as to be biased toward the lower side of the main body 110. Accordingly, free movement of the radiation gate may be possible toward the opposite side where the support 113 is not installed.

In addition, radiation gantries that move along rails may be installed on the upper side of the main body 110.

The radiation gantries 120 and 120' form a ring gate 121, and a plurality of low-dose radiation may be radiated toward the patient's body from the inner periphery of the ring gate 121.

At this time, the radiation gantries 120 and 120' may be installed in multiple units in the longitudinal direction of the main body 110, and the radiation gantries 120 and 120' may be driven in conjunction with each other or individually.

Hereinafter, the radiation gantries 120 and 120' are described. The radiation gantries 120 and 120' form the ring gate 121 which is designed to surround the patient's body. At this time, the inner periphery of the ring gate 121 is formed with multiple carbon nanotube light sources 123 that radiate therapeutic radiation toward the patient's body.

More specifically, the inner periphery of the radiation gantries 120 and 120' may be equipped with the carbon nanotube light sources 123 that emit low-energy radiation of 100~120, 120'kVp (kilovolt peak) and low-dose radiation of 10-100cGy/min, distributed by band.

At this time, the low-dose radiation (LDR) refers to radiation with low intensity and is close to natural radiation. Large amounts of radiation may be harmful to living organisms, but small amounts of low-dose radiation may actually promote the physiological activities of living organisms, extending lifespan, promoting growth, or reducing tumor incidence. This is called radiation hormesis.

Low-dose radiation in the range of miligray (mGy) has been reported to induce resistance to disease and exhibit adaptive protection effects, unlike high-dose radiation (Sakai K., et al., Dose Response, 2006, 4(4):327~332; Sakai K., et al, Int. J. Low Radiat., 2003, 1(1):142~146; Scott BR., Dose Response, 2008 6(3):299~318). According to a report, when experimental mice with diabetic genes were irradiated with a certain amount of low-dose radiation, the condition thereof was improved (Sakai K., et al., Dose Response, 2006, 4(4):327~332). Radiation of 0.25 Gy or less has the effect of protecting against DNA damage and repairing damaged DNA (Le XC., et al., Science, 1998, 280(5366):1066~1069). Low-dose radiation is known to have an immune-enhancing effect (Nogami M., et al., Int. J. Radiat. Biol., 1993, 63(6):775~783; Nogami M., et al., Radiat. Res., 1994, 139(1):47~52).

Referring to FIG. 4, the carbon nanotube light sources 123 arranged at the center of the radiation gantries 120 and 120' may be configured to emit the highest energy. The carbon nanotube light sources 123 arranged toward both ends from the center of the radiation gantries 120 and 120' may be arranged to emit gradually lower energy.

For example, as shown in FIG. 4, when 10 carbon nanotube light sources 123 are radially arranged, the carbon nanotube light sources 123 may be arranged so that a pair of carbon nanotube light sources 123, installed at two central symmetrical points, each emit 300 kV of energy, the carbon nanotube light sources 123 located on the left and right below each emit 200 KVP of energy, a pair of carbon nanotube light sources 123, symmetrical on the left and right below, each emit 100 kV of energy, a pair of carbon nanotube light sources 123, symmetrical on the left and right below, each emit 50 kV of energy, and a pair of carbon nanotube light sources 123, symmetrical on the left and right below, each emit 10 kV of energy.

When the carbon nanotube light sources 123 are arranged in the left-right symmetrical structure described above, the most suitable radiation treatment may be performed for a body structure that has a left-right symmetrical structure centered on the body. In particular, the body needs to be irradiated with relatively high energy radiation beams because the major organs are included in the body and are protected by thick subcutaneous fat and muscles. On the other hand, sufficient effects may be expected by irradiating the limbs with relatively low energy radiation beams.

In addition, the carbon nanotube light sources 123 may be formed in multiple rows on the gate inner periphery of the radiation gantries 120 and 120'. In this case, each array of carbon nanotube light sources 123 may be operated alternately.

For example, when the carbon nanotube light sources are arranged in two rows, after radiation irradiation of the carbon nanotube light sources 123 in the first row is sequentially performed, the carbon nanotube light sources 123 in the first row are in a standby state, and radiation irradiation of the carbon nanotube light sources 123 in the second row, which are waiting for treatment, is sequentially performed so that radiation treatment is performed.

In this way, by performing radiation treatment alternately using the carbon nanotube light sources 123 in rows 1 and 2, treatment time may be shortened and intensive radiation treatment may be achieved.

The radiation gantries 120 and 120' may be installed in multiple numbers in the longitudinal direction of the main body 110, and the radiation gantries 120 and 120' may be driven in conjunction with each other or individually.

For example, the radiation gantries 120 and 120' may be linked to simultaneously emit radiation concentrated on one part of a patient's body, or to simultaneously emit radiation to two or more parts of a patient's body.

In addition, among the radiation gantries 120 and 120', only specific radiation gantries 120 and 120' may be selectively operated or only specific radiation gantries 120 and 120' may be deactivated.

In addition, each of the radiation gantries 120 and 120' may cause the carbon nanotube light sources 123 of multiple rows installed in the gate inner periphery to operate alternately.

At this time, the carbon nanotube light sources 123 may be operated individually or in groups.

According to the present invention, by installing the radiation gantries 120 and 120' in multiples, continuous use is possible without a break period due to heat generated when emitting radiation beams. In addition, by allowing the radiation gantries 120 and 120' to be driven in conjunction with each other or individually, the patient's body may be irradiated alternately or simultaneously with low-dose radiation beams, which minimizes patient discomfort by minimizing waiting time in the radiation apparatus.

In addition, the present invention may meet the total radiation emission required for treatment by increasing the number of carbon nanotube light sources emitting radiation beams in a multi-gantry manner, may radiate multiple parts of the patient's body with radiation beams simultaneously by individually controlling the emission positions of radiation beams from a plurality of gantries while moving along rails, and may provide a variety of treatment options by moving the radiation beams to different parts of the body.

According to the present invention, by emitting low-dose, low-energy radiation beams through the carbon nanotube light sources, therapeutic effects may be obtained by radiating the radiation beams to targets sensitive to radiation exposure, such as the head (brain) area. In addition, uniformly radiate beams to the head (brain) area, it is necessary to increase the number of installed carbon nanotube light sources that emit radiation beams in a multi-gantry manner as in the present invention.

According to the radiation apparatus using the multi-gantry method of the present invention, a target such as the head (brain) may be uniformly irradiated with beams safely and efficiently.

In addition, according to the present invention, the durability of the carbon nanotube light sources may be prevented from deteriorating due to heat generation when emitting radiation beams by alternately operating the multiple carbon nanotube light sources, and continuous use is possible without a break period due to heat generated when emitting radiation beams.

In addition, the gantry mounting portion 130 may be formed at one end of the main body 110.

The gantry mounting portion 130 is a component that allows the radiation gantries 120 and 120' to be received in an operational standby state, and may be provided as a ring-shaped structure that surrounds the outer shape of the radiation gantries 120 and 120'.

At this time, the gantry mounting portion 130 may also be provided in a complete ring shape. As shown in FIGS. 1 to 3, the gantry mounting portion 130 may also be provided in a ring shape with one side open. At this time, a table 131 may be formed on the open end to install the controller 140.

The table 131 may be formed by extending the open end of the gantry mounting portion 130 in a horizontal direction.

At this time, the gantry mounting portion 130 may be electrically and mechanically connected to the main body 110.

However, the present invention is not limited thereto, and the gantry mounting portion 130 may be formed as an independent structure while being electrically connected to the main body 110 and mechanically separated from the main body 110. For example, this separation structure allows only the controller 140 and the gantry mounting portion 130 to be separated from the main body 110 and replaced when upgrading the radiation apparatus.

In addition, the present invention may provide the controller 140 that selectively controls the carbon nanotube light sources 123, checks the patient's condition using input patient information, sets a treatment direction, and controls the operation of the low-dose radiation apparatus 10 to perform radiation treatment.

The controller 140 may include the monitor 141 capable of monitoring patient information and an input device 143 for entering commands to control the device. At this time, the monitor 141 and the input device 143 may be replaced with a touch panel.

In the present invention, a selective treatment mode may be set according to the type of disease and the patient's condition through the controller 140.

For example, the intensity and irradiation band of radiation used in the treatment of skin cancer, benign diseases-keloids, osteoarthritis, heterotopic ossification, psoriasis, atopy, dementia, Parkinson's disease, animal cancer, acute/chronic pain in the musculoskeletal area, and intractable inflammatory diseases need to be different. Accordingly, among the carbon nanotube light sources 123 divided by band, a specific band of the carbon nanotube light sources 123 may be selectively operated, or two or more carbon nanotube light sources 123 may be operated simultaneously to perform complex treatment.

Accordingly, various treatment modes may be set in advance and operated considering the patient's age or health condition. By manipulating the controller 140, the selective operation of a plurality of carbon nanotube light sources according to the set treatment mode and the total amount and number of times of radiation irradiated may be comprehensively controlled.

In particular, when a patient lies on the bed 100 and waits for treatment, the controller 140 checks the patient's condition and sets the treatment direction through the previously entered patient information. Then, according to the judgment of the controller 140, the bed 100 is operated so that the patient is positioned in the optimal treatment position, and the radiation gantries 120 and 120' are operated so that radiation treatment is performed.

By installing the controller 140 on the outside of the gantry mounting portion 130, an operator may easily operate the controller 140.

The carbon nanotube light sources 123 used in the present invention may include a metal emitter and a carbon nanotube X-ray source including carbon nanotubes (CNTs) grown from the emitter.

At this time, the carbon nanotube (CNT) emits electrons in a field emission manner and may be formed in an irregular shape facing upward on the top of a pad. The growth process of CNTs may be performed through plasma enhanced chemical vapor deposition (PECVD) or thermal chemical vapor deposition (thermal CVD) that supplies hydrocarbon gas.

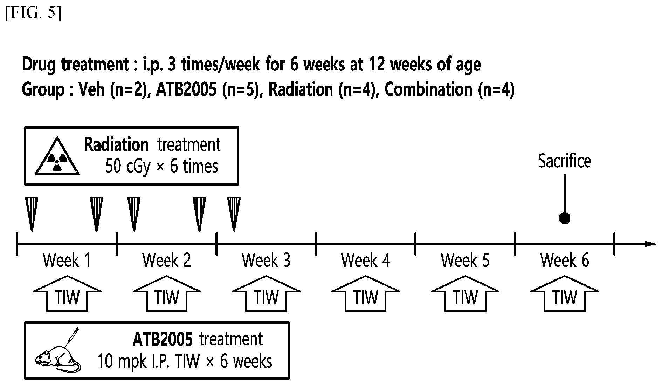

FIG. 5 is a graph showing the progress of concurrent drug treatment and radiation treatment performed on experimental mice according to the conventional method.

Referring to FIG. 5, when drug treatment, radiation treatment, and ATB2005 treatment were performed concurrently on 12-week-old experimental mice according to the conventional method, the experimental mice survived until the sixth week. At this time, drug treatment was performed three times a week for six weeks, radiation treatment was performed five times at 50 cGy, and ATB2005 treatment was performed for six weeks under the condition of 10mpk I.P. TIW.

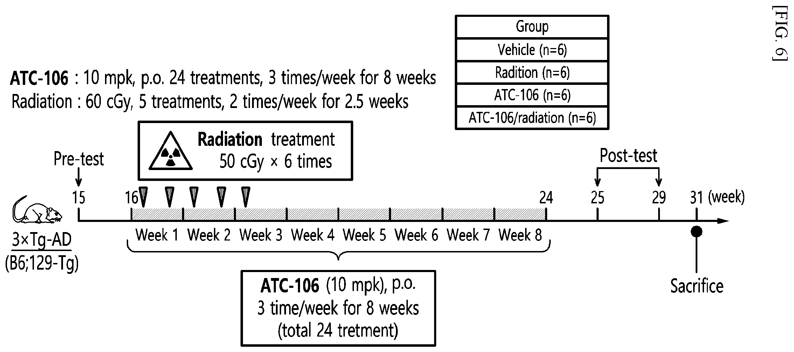

FIG. 6 is a graph showing the progress of concurrent treatment on experimental mice using the low-dose radiation apparatus of the present invention.

Referring to FIG. 6, according to the low-dose radiation apparatus of the present invention, when experiments were conducted on 12-week-old laboratory mice, ATC-108 (10 mpk) treatment was performed 24 times in total at 3-week intervals for 8 weeks, and radiation treatment was performed 5 times in total under conditions of low-dose radiation of 100-300 kVp, 60 cGy at 2-week intervals for 2.5 weeks, the experimental mice survived until the 31st week.

When comparing the experimental results (6 weeks survival) according to the conventional method of FIG. 5 and the experimental results (31 weeks survival) according to the low-dose radiation apparatus of the present invention of FIG. 6, it can be confirmed that the survival rate of mice is far superior in the low-dose radiation apparatus of the present invention.

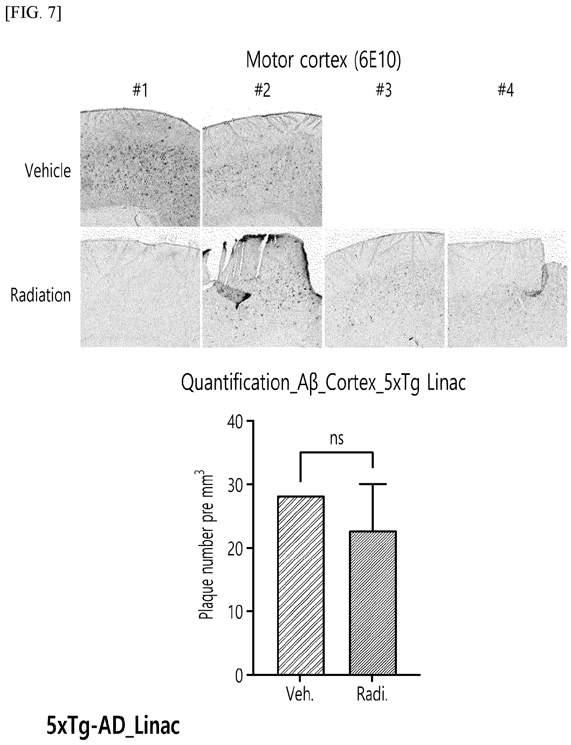

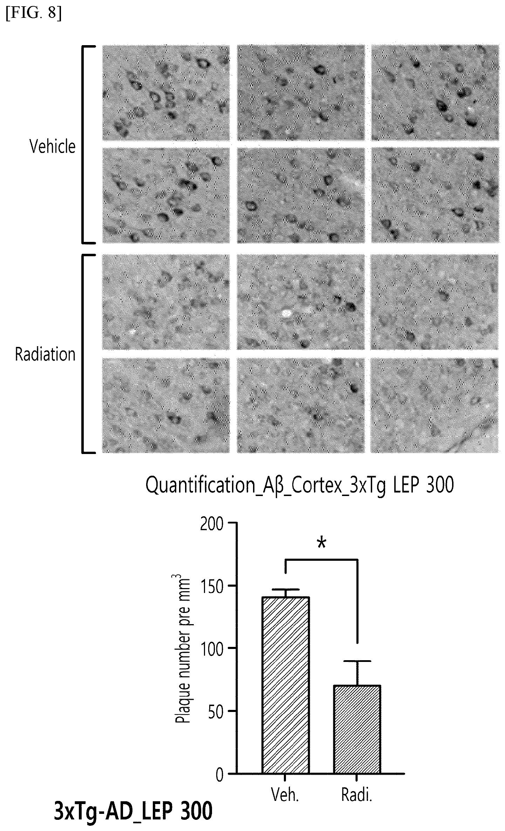

FIG. 7 is a graph comparing Aβ cortical images and the number of plaques per unit area (mm²) according to the results of radiation treatment of the conventional method with the control group, and FIG. 8 is a graph comparing Aβ cortical images and the number of plaques per unit area (mm²) according to the results of low-dose radiation treatment of the present invention with the control group.

Referring to FIGS. 7 and 8, FIG. 7 shows the treatment results using conventional 6MeV radiation. According to the results, compared to the control group (Vehicle) that did not receive radiation treatment, it was confirmed that the number of plaques per unit area (mm²) was 28:22, which was not significantly reduced.

In contrast, FIG. 8 shows the results of low-dose radiation treatment of the present invention, that is, treatment results using 300 kVp radiation. According to the results, compared to the control group (Vehicle) that did not receive radiation treatment, it was confirmed that the number of plaques per unit area (mm²) was 140:70, which was significantly reduced.

As a result of comparison with the control group in FIGS. 7 and 8, the reduction in the number of plaques per area (mm²) was greater in the low-dose radiation kVp unit according to the present invention than in the case of radiation MeV unit according to the conventional method. At this time, the number of plaques is measured by staining cells for amyloid beta (Aβ), and is proportional to the degree of dementia.

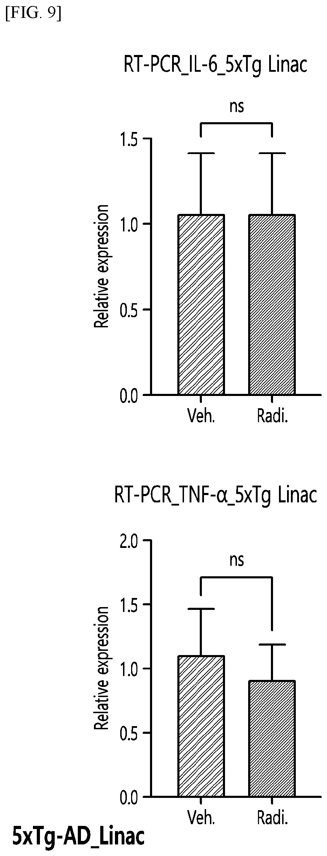

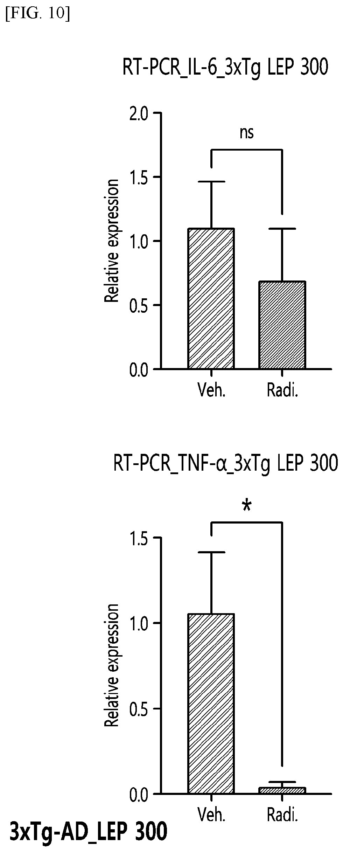

FIG. 9 is a graph showing the relative expression of radiation treatment results according to the conventional method compared to the control group through RT-PCR, and FIG. 10 is a graph showing the relative expression of low-dose radiation treatment results according to the present invention compared to the control group through RT-PCR.

As shown in FIG. 9, for the results of conventional radiation treatment, RT-PCR_TNF-a and RT-PCR_IL-6 were performed respectively. As a result, the relative expression ratios with the control group were 1: 1 and 1:0.8, respectively, showing that there was almost no difference in the ratio.

As shown in FIG. 10, for the results of low-dose radiation treatment of the present invention, RT-PCR_TNF-a and RT-PCR_IL-6 were performed respectively. As a result, the relative expression ratios with the control group were 1.2:0.6 and 1:0.1, respectively, showing that the ratio difference is more than twice.

Recently, several preclinical and exploratory clinical studies have reported that low-dose radiation has anti-inflammatory and neuroprotective effects, which are explained by the mechanism of inhibiting the NFkb pathway and regulating microglial cell phenotype.

A study reported that among 4 patients with advanced dementia who underwent 120kVp X-ray energy (brain computed tomography CT) 3 times for a total of 16cGy, 3 showed remarkable improvement in cognitive function (75%) (reference: low dose of ionizing radiation as a treatment for alzheimer's disease : journal of alzheimer's disease 2021).

In addition, a study reported that when radiation in the region where the Compton effect (6 MeV) is dominant was used in patients with osteoarthritis, the pain reduction treatment effect was 13 to 21% superior when 120 to 300 kVp, where the photoelectric effect is dominant, was used (reference: Strahlentherapie und Onkologie volume 196, pages715??724 (2020).

Previous studies have reported that when the same dose of radiation is applied, telomere shortening is not significantly affected by the quality of radiation (electrons, protons, alpha particles), but the biological effects on chromosome abberation and cells (apoptosis) are significantly greater in high LET protons and heavy ions than in low LET radiation.

Accordingly, when comparing the biological effects of radiation when using radiation in the range of 120-300kVp where the photoelectric effect is dominant and radiation in the range of Compton effect (6MeV) where the Compton effect is dominant, the radiobiologic effect (RBE, (radiation biological effect) is twice as large at 120 to 300 kVp compared as at 6 MeV (2 to 1). In addition, it can be predicted that the energy of 120 to 300 kVp, where energy decays rapidly, is more effective in biological effects in the low-dose range, especially in cell protection and anti-inflammatory effects.

As described above, according to the present invention, by installing multiple gantries, continuous use is possible without a break period due to heat generated when emitting radiation beams. In addition, by allowing the gantries to be driven individually, the patient's body may be irradiated with low-dose radiation beams alternately or simultaneously. **In** addition, the present invention may provide a variety of treatment options by irradiating multiple parts of the patient's body with radiation beams simultaneously by individually controlling the emission positions of radiation beams from a plurality of gantries while moving along rails, or by moving the radiation beams to different parts of the body. Accordingly, the present invention may provide an optimized radiation treatment environment by adjusting the energy size, angle, and dose of radiation radiated from the radiation apparatus according to treatment purposes and individual patient characteristics.

Although the present invention has been described with reference to limited embodiments and drawings, it should be understood by those skilled in the art that various changes and modifications may be made therein. For example, the described techniques may be performed in a different order than the described methods, and/or components of the described systems, structures, devices, circuits, etc., may be combined in a manner that is different from the described method, or appropriate results may be achieved even if replaced by other components or equivalents.

Therefore, other embodiments, other examples, and equivalents to the claims are within the scope of the following claims.

## Claims

1. A multi-gantry low-dose radiation apparatus, comprising:
a main body having a bed on which a patient lies and receives treatment, wherein the bed is formed longitudinally on the main body;
radiation gantries installed to radiate a plurality of low-dose radiation toward a patient's body from an inner periphery of a ring gate while moving along rails on an upper side of the main body;
a gantry mounting portion formed at one end of the main body to accommodate the radiation gantries in an operational standby state; and
a controller installed on one side of the gantry mounting portion to check a condition of a patient and set a treatment direction using entered patient information and control operation of the radiation gantries,
wherein the radiation gantries are installed in multiple numbers along a length of the main body and are driven in conjunction with each other or individually.

2. The multi-gantry low-dose radiation apparatus according to claim 1, wherein the bed on which a patient lies and receives treatment is formed longitudinally on an upper part of the main body, the main body is provided with an open structure, and rails are formed on sides of the main body to guide movement of the radiation gantries.

3. The multi-gantry low-dose radiation apparatus according to claim 2, wherein a support is formed on one lower side of the main body to secure the main body in a state of floating at a certain height from a floor of a building, wherein the support is installed in a biased position on one lower side of the main body, allowing free movement of the radiation gate to an opposite side where the support is not installed.

4. The multi-gantry low-dose radiation apparatus according to claim 1, wherein the radiation gantries are installed in multiple numbers in a longitudinal direction of the main body, and the radiation gantries are driven in conjunction with each other or individually.

5. The multi-gantry low-dose radiation apparatus according to claim 4, wherein the radiation gantries form a ring gate that surrounds a body of a patient, a plurality of carbon nanotube light sources that radiate therapeutic radiation toward the body of the patient are formed at an inner periphery of the ring gate, and the carbon nanotube light sources emitting low-energy of 100 to 120, 120'kVp (kilovolt peak) and low-dose radiation of 10 to 100 cGy/min are distributed according to bands.

6. The multi-gantry low-dose radiation apparatus according to claim 5, wherein the carbon nanotube light sources placed at a center of the radiation gantries emit the highest energy, the carbon nanotube light sources placed toward both ends centered on the center of the radiation gantries emit progressively lower energy, and the carbon nanotube light sources are arranged in a left-right symmetrical structure.

7. The multi-gantry low-dose radiation apparatus according to claim 6, wherein the carbon nanotube light sources are formed in multiple arrays on a gate inner periphery of the radiation gantries, and each array of carbon nanotube light sources is operated alternately.

8. The multi-gantry low-dose radiation apparatus according to claim 1, wherein the gantry mounting portion is provided as a ring-shaped structure that surrounds an outer surface of the radiation gantries to accommodate the radiation gantries in an operational standby state, and a table for installing a controller is formed at an open end thereof.

9. The multi-gantry low-dose radiation apparatus according to claim 8, wherein the gantry mounting portion is electrically and mechanically or electrically connected to the main body, and is formed as an independent structure that is mechanically separated from the main body.

10. The multi-gantry low-dose radiation apparatus according to claim 1, wherein the controller comprises a monitor for monitoring patient information and an input device for entering commands to control the apparatus.

11. The multi-gantry low-dose radiation apparatus according to claim 10, wherein the controller is provided as a touch panel.
